(19) Europäisches Patentamt

European Patent Office

Office européen des brevets

(11) Veröffentlichungsnummer: **0 248 401 B1**

(12) **EUROPÄISCHE PATENTSCHRIFT**

(45) Veröffentlichungstag der Patentschrift: **10.02.93**

(21) Anmeldenummer: **87107961.2**

(22) Anmeldetag: **02.06.87**

(51) Int. Cl.⁵: **C12N 9/02**, C12P 7/60

(54) **Enzym und Verfahren zu seiner Herstellung.**

(30) Priorität: **03.06.86 GB 8613429**
**14.04.87 GB 8708908**

(43) Veröffentlichungstag der Anmeldung:
**09.12.87 Patentblatt 87/50**

(45) Bekanntmachung des Hinweises auf die
Patenterteilung:
**10.02.93 Patentblatt 93/06**

(84) Benannte Vertragsstaaten:
**AT BE CH DE FR GB IT LI NL**

(56) Entgegenhaltungen:
**EP-A- 0 213 591**
**DE-A- 2 343 587**

**BIOTECHNOLOGY AND BIOENGINEERING,
Band 17, Nr. 10, 1975, Seiten 1485-1514, John
Wiley & Sons, Inc.; S. MAKOVER et al.: "New
mechanisms for the biosynthesis and metabolism of 2-Keto-L-Gulonic acid in bacteria"**

**EUROPEAN JOURNAL APPLIED MICROBIO-
LOGY, Band 2, Nr. 1, Seiten 1-8, Springer-
Verlag; I. KITAMURA et al.: "Metabolism of
L-Sorbose by Enzymes from Gluconobacter
melanogenus IFO 3293"**

(73) Patentinhaber: **F. HOFFMANN-LA ROCHE AG
Postfach 3255
CH-4002 Basel(CH)**

(72) Erfinder: **Fujiwara, Akiko
Fueta 1059-7
Kamakura-shi Kanagawa-ken(JP)**
Erfinder: **Hoshino, Tatsuo
Fueta 808-47,
Kamakura-shi Kanagawa-ken(JP)**
Erfinder: **Sugisawa, Teruhide
Kohnandai 8-26-10 Kohnan-ku
Yokohama-shi Kanagawa-ken(JP)**

(74) Vertreter: **von Hellfeld, Axel, Dr. Dipl.-Phys. et
al
Wuesthoff & Wuesthoff Patent- und Rechtsanwälte Schweigerstrasse 2
W-8000 München 90(DE)**

**Beschreibung**

Die Erfindung betrifft die neue L-Sorboson-Dehydrogenase und ein Verfahren zu deren Herstellung.

Die erfindungsgemässe L-Sorboson-Dehydrogenase katalysiert die Oxidation von L-Sorboson zu 2-Keto-L-gulonsäure (im folgenden als 2-KGA bezeichnet) in Gegenwart von Nicotinamidadenindinukleotid (im folgenden als NAD bezeichnet) oder Nicotinamidadenindinukleotidphosphat (im folgenden als NADP bezeichnet) als Coenzym. 2-KGA ist ein wichtiger Vorläufer bei der Herstellung von Vitamin C.

Eine Reaktion zur mikrobiellen Umwandlung von L-Sorboson in 2-KGA ist bekannt. Die Herstellung von 2-KGA aus L-Sorboson unter Verwendung von zellfreien Extrakten von Mikroorganismen ist aus verschiedenen Publikationen bekannt geworden. Gemäss der US-Patentschrift Nr. 3.907.639 ermöglichen die zu den Gattungen Acetobacter, Pseudomonas, Escherichia, Serratia, Bacillus, Staphylococcus, Aerobacter, Alcaligenes, Penicillium, Candida und Gluconobacter gehörenden Mikroorganismen eine solche Umwandlung. Ferner berichten Kitamura et al. [Europ. J. Appl. Microbiol., 2, 1 (1975)], dass das L-Sorboson oxidierende, in Gluconobacter melanogenes IFO 3293 gefundene Enzym weder ein Coenzym noch einen Elektronenakzeptor zur Entwicklung von Enzymaktivität benötige. Makover et al. [Biotechnol. Bioeng. 17, 1485 (1975)] berichten über das Vorhandensein von L-Sorboson-Dehydrogenase-Aktivität in der partikulären Fraktion von Pseudomonas putida ATCC 21812 und Gluconobacter melanogenus IFO 3293. Diese meinen weiter, dass Nicotinamidadenindinukleotid oder Nicotinamidadenindinukleotidphosphat nicht als Coenzym für das Enzym diene.

Wie oben gesagt, verlautet bisher nichts über ein gereinigtes Enzym, das die Aktivität zur Oxidation von L-Sorboson zu 2-KGA in Gegenwart von NAD oder NADP als Coenzym aufweist.

Es wurde nun gefunden, dass das aus der Cytosol-Fraktion von Zellen bestimmter Mikroorganismen isolierte, gereinigte Enzym die Oxidation von L-Sorboson zu 2-KGA katalysiert. Die vorliegende Erfindung beruht auf diesem Befund.

Gegenstand der vorliegenden Erfindung ist die neue L-Sorboson-Dehydrogenase, die die Oxidation von L-Sorboson zu 2-KGA in Gegenwart von NAD oder NADP als Coenzym katalysiert. Ein weiterer Gegenstand ist ein Verfahren zur Herstellung der neuen L-Sorboson-Dehydrogenase durch Kultivieren eines zur Gattung Gluconobacter gehörenden Mikroorganismus oder einer Mutante davon, die die Herstellung der neuen L-Sorboson-Dehydrogenase in Zellmaterial ermöglichen, Zertrümmern des Zellmaterials, Isolieren der L-Sorboson-Dehydrogenase aus zellfreiem Extrakt des zertrümmerten Zellmaterials, vorzugsweise aus der Cytosol-Fraktion des Zellmaterials, sowie Reinigen der L-Sorboson-Dehydrogenase.

Die physiko-chemischen Eigenschaften der gereinigten neuen L-Sorboson-Dehydrogenase, deren Herstellung in den nachfolgenden Beispielen veranschaulicht wird, sind wie folgt:

(1) Enzymaktivität

Die L-Sorboson-Dehydrogenase der vorliegenden Erfindung katalysiert die Oxidation von L-Sorboson zu 2-KGA in Gegenwart von NAD oder NADP als Coenzym gemäss der folgenden Reaktionsgleichung.

L-Sorboson + NAD oder NADP → 2-KGA + reduziertes NAD oder NADP

- Test zur Bestimmung der Enzymaktivität

Die Enzymaktivität wurde durch Bestimmung der Absorptionszunahme des reduzierten NAD oder NADP bei 340 nm gemessen. Das Reaktionsgemisch (0,41 ml) enthielt 1,0 mg L-Sorboson, 0,2 mg NAD in 50 mM Kaliumphosphatpuffer (pH-Wert 7,0) und das Enzym. Die Reaktion wurde durch Zugabe des Enzyms in Gang gebracht, und die Geschwindigkeit der NAD- oder NADP-Bildung wurde unter Verwendung eines Spektrophotometers (UVIKON 810 Kontron K.K.) bei 30°C gemessen. Unter einer Einheit Enzymaktivität ist diejenige Menge Enzym, die die Bildung von 1 μMol von reduziertem NAD oder NADP pro Minute bei 30°C katalysiert, zu verstehen.

- Coenzym-Bedarf

Unter Verwendung der Standardtestbedingung wurde der Bedarf an Coenzym für das Auftreten von Enzymaktivität untersucht. Aus der Tabelle 1 geht hervor, dass sowohl NAD als auch NADP als Coenzym dienen können.

## Tabelle 1

| Coenzym | Relative Aktivität (%) |
|---------|------------------------|
| ohne    | 0                      |
| NAD     | 100                    |
| NADP    | 35,7                   |

(2) Substratspezifität

Die Substratspezifität des Enzyms wurde unter Verwendung einer Standardenzymtestmethode bestimmt. Diese Methode unterscheidet sich von der unter (1) oben beschriebenen Methode dadurch, dass je 1 mg der verschiedenen Substrate anstelle von L-Sorboson verwendet wurde. Die Ergebnisse der Bestimmung sind in der Tabelle 2 aufgeführt. L-Sorboson war das bestgeeignete Substrat für dieses Enzym. Glyoxal, Glykolaldehyd, Glutaraldehyd, Propionaldehyd, Methylglyoxalacetaldehyd und D-Mannose wurden auch oxidiert.

## Tabelle 2

| Substrat         | Relative Aktivität (%) |
|------------------|------------------------|
| L-Sorboson       | 100                    |
| Glyoxal          | 33,3                   |
| Glykolaldehyd    | 23,3                   |
| Glutaraldehyd    | 16,7                   |
| Propionaldehyd   | 13,3                   |
| Methylglyoxal    | 10,0                   |
| Acetaldehyd      | 8,0                    |
| D-Mannose        | 4,9                    |
| Benzaldehyd      | 0                      |
| Glyoxylsäure     | 0                      |

| | |
|---|---|
| Glykolsäure | 0 |
| D-Glucoson | 0 |
| D-Fructose | 0 |
| D-Glucose | 0 |
| L-Sorbose | 0 |
| Dihydroxyaceton | 0 |
| Hydroxybrenztraubensäure | 0 |
| L-Glyceroson | 0 |

(3) Optimaler pH-Wert

Das Verhältnis zwischen der Reaktionsgeschwindigkeit der L-Sorboson-Dehydrogenase und dem pH-Wert wurde in Kaliumphosphatpuffer und Ammoniumpuffer verschiedener pH-Werte bestimmt. Die Ergebnisse sind in der Tabelle 3 aufgeführt. Das Enzym wies die grösste Enzymaktivität bei einem pH-Wert von ca. 9,0 auf.

## Tabelle 3

| pH-Wert | Puffer (50 mM) | Relative Aktivität (%) |
|---|---|---|
| 6,0 | Kaliumphosphat | 34,7 |
| 6,5 | | 71,4 |
| 7,0 | | 100,0 |
| 7,5 | | 163,3 |
| 8,0 | | 269,4 |
| 8,0 | $NH_4OH/NH_4Cl$ | 93,9 |
| 9,0 | | 326,5 |
| 10,0 | | hohe Hintergrundaktivität |

(4) Stabilität bei verschiedenen pH-Werten

Das gereinigte Enzym wurde zu McIlavaine-Puffer (Gemisch von 0,1M Zitronensäure und 0,2M Dinatriumphosphat) verschiedener pH-Werte gegeben, und das Gemisch wurde 24 Stunden bei 4°C stehengelassen. Die Restaktivität wurde unter den Standardtestbedingungen, wie diese oben unter (1) beschrieben sind, getestet. Die Ergebnisse der Bestimmung sind in der Tabelle 4 aufgeführt. Das gereinigte Enzym war zwischen den pH-Werten 6,0 und 8,0 relativ stabil.

4

EP 0 248 401 B1

## Tabelle 4

| pH-Wert | Relative Aktivität (%) |
|---------|------------------------|
| 4 | 61,6 |
| 5 | 76,8 |
| 6 | 88,9 |
| 7 | 100 |
| 8 | 94,9 |

(5) Wärmestabilität

Das gereinigte Enzym wurde 10 Minuten bei verschiedenen Temperaturen mit 10mM Kaliumphosphatpuffer (pH-Wert 7,0) versetzt und danach in Eis/Wasser abgekühlt. Die Restaktivität wurde unter den Standardtestbedingungen, wie diese oben unter (1) beschrieben sind, gemessen. Die Ergebnisse sind in der Tabelle 5 aufgeführt. Das gereinigte Enzym war bis 50°C stabil und verlor nach Inkubation bei 60°C etwa 80% seiner Aktivität.

## Tabelle 5

| Behandlung | Relative Aktivität (%) |
|------------|------------------------|
| 20°C 10 Minuten | 100 |
| 30°C " | 96,4 |
| 40°C " | 92,9 |
| 50°C " | 82,1 |
| 60°C " | 21,4 |

(6) Optimale Temperatur

Die Enzymaktivität von L-Sorboson-Dehydrogenase wurde bei Temperaturen von 25°C bis 60°C unter den Standardtestbedingungen, wie diese oben unter (1) beschrieben sind, bestimmt. Die Ergebnisse sind in der Tabelle 6 aufgeführt. Die Aktivität des erfindungsgemässen Enzyms nimmt mit steigender Temperatur, und zwar bis 60°C zu.

5

## Tabelle 6

| Temperatur (°C) | Relative Aktivität (%) |
|---|---|
| 25 | 62,5 |
| 30 | 100 |
| 35 | 143 |
| 40 | 180 |
| 45 | 225 |
| 50 | 256 |
| 55 | 287 |
| 60 | 312 |

(7) Molekulargewicht

Die Enzymlösung wurde auf eine TSK-Gel G4000SW (Toyo Soda Co., Ltd.)-HPLC-Säule, die mit 10mM Kaliumphosphatpuffer (pH-Wert 7,0) äquilibriert worden war, aufgetragen und mit demselben Puffer eluiert. Die Enzymaktivität wurde in denjenigen Fraktionen festgestellt, die einem Molekulargewicht von 190.000 ± 20.000 entsprachen.

(8) Bestimmung des Km-Wertes

Die Geschwindigkeit der Oxidation unter variierender Konzentration von NAD oder NADP wurde unter den Standardtestbedingungen bestimmt, um die Km-Werte in Gegenwart von überschüssigem L-Sorboson festzustellen. Für die scheinbare Michaelis-Konstante (Km) für NAD und NADP wurden Werte von $4,55 \times 10^{-5}$ M und $1,06 \times 10^{-5}$ M berechnet.

(9) Einfluss von Metallionen

Unter Verwendung der oben beschriebenen Testmethode wurde der Einfluss von verschiedenen Metallionen auf die Enzymaktivität untersucht. Die Ergebnisse der Bestimmung sind in der Tabelle 7 aufgeführt. $Mg^{2+}$ und $Fe^{2+}$ wirkten stimulierend, und $Cu^{2+}$ hemmend.

Tabelle 7

| Metallion | Konzentration (µM) | Relative Aktivität (%) |
|-----------|-------------------|------------------------|
| ohne | – | 100 |
| $Mg^{2+}$ | 10 | 113 |
| $Fe^{2+}$ | 1 | 116 |
| $Mo^{2+}$ | 10 | 96 |
| $Cu^{2+}$ | 0,1 | 0 |

(10) Einfluss von Hemmstoffen

Unter Verwendung der oben beschriebenen Testmethode wurde der Einfluss verschiedener Hemmstoffe auf die Enzymaktivatät untersucht. Die Ergebnisse sind in der Tabelle 8 aufgeführt. N-Aethylmaleimid hemmte die Enzymaktivität ausgesprochen.

Tabelle 8

| Substanz | Konzentration (mM) | Relative Aktivität (%) |
|----------|-------------------|------------------------|
| N-Aethylmaleimid | 1 | 0 |
| $JCH_2COONa$ | 5 | 57,7 |
| $NaN_3$ | 5 | 100 |
| Dithiothreitol | 5 | 119,2 |
| – | – | 100 |

(11) Reinigung

Die Reinigung von L-Sorboson-Dehydrogenase kann mittels bekannten Reinigungsmethoden bzw. durch Kombination bekannter Reinigungsmethoden durchgeführt werden, z.B. Ionenaustauscherchromatographie, Flüssigkeitschromatographie, Gelfiltration, Gelelektrophorese, Aussalzen und Dialyse.

Die erfindungsgemässe L-Sorboson-Dehydrogenase kann durch Kultivieren eines entsprechenden Mikroorganismus, Zertrümmern der Zellen und Isolieren und Reinigen der L-Sorboson-Dehydrogenase aus zellfreiem Extrakt zertrümmerten Zellmaterials, vorzugsweise aus der Cytosol-Fraktion des Zellmaterials, hergestellt werden.

Die im Rahmen der vorliegenden Erfindung verwendeten Mikroorganismen gehören der Gattung Gluconobacter oder Mutanten davon an. Nach neuester Klassifikation fallen sämtliche Gluconobacter Stämme unter die Spezies Gluconobacter oxydans. Die morphologischen und physiologischen Kennzeichen der Stämme, die Gluconobacter oxydans angehören, sind in "Bergey's Manual of Systematic Bacteriology", Vol. I, 275-278 (1984) und F. Gossele et al., International J. System. Bacteriol. Vol. 33, 65-81 (1983) beschrieben.

Der Gattung Gluconobacter angehörende Mikroorganismen, die im Rahmen der vorliegenden Erfindung verwendet werden, können aus natürlichen Quellen isoliert werden bzw. sind bei Kultursammlungen erhältlich. Auch derivierte Mutanten können gemäss der vorliegenden Erfindung verwendet werden.

Die in der vorliegenden Erfindung verwendeten Mutanten können erhalten werden durch Behandlung eines Wildtyp-Stammes mit einem Mutagen, wie durch UV-Bestrahlung, Röntgenbestrahlung, $\gamma$-Bestrahlung oder durch Behandlung mit salpetriger Säure oder anderen geeigneten Mutagenen oder durch Isolierung eines Klons, der durch spontane Mutation auftritt. Diese Mutationen eines Wildtyp-Stammes oder eines Mutantenstammes davon können auf an sich bekannte Weise durchgeführt werden. Viele dieser Methoden sind in der Literatur beschrieben, z.B. "Chemical Mutagens", Herausgeber Y. Tajima, T. Yoshida und T. Kada, Kodansha Scientific Inc., Tokyo, Japan, 1973.

Die erfindungsgemässen Mutanten sind auch durch Fusion der zur Spezies Gluconobacter oxydans gehörenden Stämme und durch Kombination von Mutagenese und/oder Fusion zugänglich.

Beispiele von Stämmen, die bevorzugt verwendet werden, sind Gluconobacter oxydans UV-10, Gluconobacter oxydans E-1, Gluconobacter oxydans H-2 und Gluconobacter oxydans L-8. Diese Mikroorganismen sind bei der Agency of Industrial Science and Technology, Fermentation Research Institute, Japan unter den folgenden Hinterlegungsnrn. deponiert:

|  |  | **Zugriffs-Nr.** |
|---|---|---|
| Gluconobacter oxydans UV-10 | FERM-P Nr. 8422, | FERM BP-1267 |
| Gluconobacter oxydans E-1 | FERM-P Nr. 8353, | FERM BP-1265 |
| Gluconobacter oxydans H-2 | FERM-P Nr. 8354, | FERM BP-1266 |
| Gluconobacter oxydans L-8 | FERM-P Nr. 8355, | FERM BP-1268 |

Die Mikroorganismen können in wässrigem Medium, das die geeigneten Nährstoffe enthält, unter aeroben Bedingungen kultiviert werden. Die Kultivierung kann, z.B. bei einem pH-Wert von 4,0 bis ca. 8,0, vorzugsweise bei 4,5 bis 6,5, durchgeführt werden. Die Kultivierungszeit hängt von der Natur der Mikroorganismen und dem verwendenteten Nährstoff ab, beträgt vorzugsweise ca. 10 bis 100 Stunden. Der bevorzugte Temperaturbereich zur Durchführung der Kultivierung beträgt ca. 10-40°C, insbesondere 25-35°C.

Das Kulturmedium enthält z.B. folgende Nährstoffe: assimilierbare Kohlenstoffquellen, wie Glycerin, D-Mannit, D-Sorbit, Erythrit, Ribit, Xylit, Arabit, Inosit, Dulcit, D-Ribose, D-Fructose, D-Frucose, D-Glucose, Gluconat, L-Sorbose, Maltose und Sucrose, vorzugsweise L-Sorbose, D-Sorbit oder Glycerin; abbaubare Stickstoffquellen, wie organische Substanzen, z.B. Pepton, Hefeextrakt, Sojabohnenmehl und Maisquellwasser; und anorganische Substanzen, z.B. Ammoniumsulfat, Ammoniumchlorid und Kaliumnitrit; Vitamine und Spurenelemente.

Im folgenden wird eine Ausführungsform für die Isolierung von L-Sorboson-Dehydrogenase aus den Mikroorganismen nach der Kultivierung und die Reinigung der L-Sorboson-Dehydrogenase beschrieben:

(1) Zellen werden aus der Fermentationsbrühe durch Zentrifugation geerntet.

(2) Die geernteten Zellen werden mit Wasser, physiologischer Kochsalzlösung oder einer Pufferlösung mit einem geeigneten pH-Wert gewaschen.

(3) Die gewaschenen Zellen werden in der Pufferlösung suspendiert und durch einen Homogenisator, ein Ultraschallgerät oder durch Behandlung mit Lysozym und dergleichen zertrümmert.

(4) L-Sorboson-Dehydrogenase wird aus dem zellfreien Extrakt des zertrümerten Zellmaterials isoliert und gereinigt, vorzugsweise aus der Cytosol-Fraktion des Zellmaterials.

Die erfindungsgemässe L-Sorboson-Dehydrogenase eignet sich als Katalysator zur Herstellung von 2-KGA aus L-Sorboson. Die Reaktion sollte bei pH-Werten von ca. 5,0 bis ca. 10,0 in Gegenwart von NAD oder NADP in einer Lösung, wie Phosphatpuffer, Ammoniumpuffer und dergleichen, durchgeführt werden. Ein bevorzugter Temperaturbereich zur Durchführung der Reaktion beträgt ca. 20°C bis ca. 70°C. Die besten Ergebnisse werden bei pH-Werten im Bereich ca. 8,0-9,0 und bei 60°C erzielt. Die Konzentration von L-Sorboson in einer Lösung hängt von anderen Reaktionsbedingungen ab, liegt jedoch vorzugsweise zwischen 10 und 100 g/l, insbesondere zwischen 30 und 40 g/l.

Das Enzym kann auch im immobilisierten Zustand auf einem geeigneten Träger verwendet werden. Das Enzym kann auf irgendeine gängige Weise immobilisiert werden. Beispielsweise kann das Enzym unmittelbar an eine Membran oder an ein Harz, welche(s) funktionelle Gruppen aufweist, gebunden werden, oder es kann durch als Brücken dienenden Verbindungen, die bifunktionelle Gruppen aufweisen, z.B. Glutaraldehyd, an das Harz gebunden werden.

Die nachfolgenden Beispiele veranschaulichen die vorliegende Erfindung.

Beispiel 1

Herstellung von L-Sorboson-Dehydrogenase

(1) Kultivierung von Gluconobacter oxydans UV-10 (FERM-P Nr. 8422)

Eine Agarschrägkultur von Gluconobacter oxydans UV-10 (FERM-P Nr. 8422) wurde in 5 ml des Medium Nr. 3B in einem Reagenzglas inokuliert und bei 27°C innert 3 Tagen in einer Reagenzglas-Schüttelvorrichtung kultiviert. Das Medium enthielt 7,0% L-Sorbose, 0,05% Glycerin, 1,5% Hefeextrakt (Oriental Co.), 0,25% Magnesiumsulfat-heptahydrat und 1,0% Calciumcarbonat. 1 ml dieser Kultur wurde in 50 ml des gleichen Mediums in einem 500 ml-Erlenmeyer-Kolben übertragen und bei 27°C innert 3 Tagen in einer Schüttelvorrichtung (180 Upm) kultiviert. 800 ml der so hergestellten Kultur wurden als Inoculum für einen 30 l-Fermenter verwendet, der 20 l des Mediums Nr. 3B enthielt. Der Fermenter wurde bei 30°C, 400 Upm sowie 1 Vvm (Volumen Luft/Volumen Medium/Minute) für Belüftung verwendet. Nach 40 Stunden Fermentation wurden die Zellen geerntet. Die Brühe wurde bei 1500 Upm (365 x g) 10 Minuten zentrifugiert, um das Calciumcarbonat abzutrennen, anschliessend bei 8000 Upm (10000 x g), um die Zellen zu sedimentieren. Der Zellkuchen wurde mit 0,85% wässriger Natriumchloridlösung einmal gewaschen. Aus 20 l Brühe wurden 374 g (Nettogewicht) Zellen erhalten.

(2) Herstellung von Cytosolfraktion

Die Zellen von Gluconobacter oxydans (94 g) der vorhergehenden Stufe (1) wurden zweimal mit physiologischer Kochsalzlösung gewaschen. Die gewaschenen Zellen wurden in 470 ml 10mM Kaliumphosphatpuffer suspendiert und mittels eines Dyno Mühle-Homogenisators (A. Willy, Bachofen Co., Basel) in Gegenwart von Glaskügelchen (0,1 mm ∅) bei 2000 Upm 4 Minuten zertrümmert. Zelltrümmer wurde durch 10-minütige Zentrifugation bei 4000 Upm (1,800 x g) entfernt, und anschliessend wurde der zellfreie Extrakt bei 40.000 Upm (80.000 x g) 1 Stunde zentrifugiert. Der resultierende Ueberstand wurde als Cytosol-Fraktion (470 ml) abgetrennt.

(3) DEAE Sepharose CL-6B-Säulenchromatographie

Sämtliche Verfahrensschritte wurden bei 4°C durchgeführt. 470 ml der Cytosol-Fraktion von UV-10 wurden gegen 10mM Kaliumphosphatpuffer (pH-Wert 7,0) 15 Stunden dialysiert. Das dialysierte Cytosol wurde auf eine DEAE Sepharose CL-6B-Säule (3,8 ∅ x 25 cm) aufgetragen, die mit demselben Puffer äquilibriert worden war. Nachdem die Säule mit dem Puffer gewaschen worden war, eluierte man das Enzym mittels linearem Gradienten (0 bis 0,2M) von Natriumchlorid in demselben Puffer. Die Enzymaktivität wurde in denjenigen Fraktionen nachgewiesen, die mit ca. 0,1M von Natriumchlorid eluiert worden waren. Anschliessend wurden die aktiven Fraktionen vereinigt und gegen 10mM Kaliumphosphatpuffer (pH-Wert 7,0) innert 5 Stunden dialysiert.

(4) DEAE Sephadex A-50-Säulenchromatographie

Die dialysierte Lösung wurde an einer DEAE Sephadex-A-50-Säule (2,5 ∅ x 13 cm) chromatographiert, die mit demselben Puffer äquilibriert worden war. Die Säule wurde mit dem Puffer gewaschen, und die Elution des Enzyms wurde mit einem linearen Gradienten von Natriumchlorid bis zu 0,2M in demselben Puffer durchgeführt. Die aktiven Fraktionen wurden vereinigt und gegen 10mM Kaliumphosphatpuffer (pH-Wert 7,0) dialysiert.

(5) Blue Sepharose CL-6B-Säulenchromatographie

Die dialysierte Lösung wurde auf eine Blue Sepharose CL-6B-Säule (1,5 ∅ x 10 cm) aufgetragen. Nachdem die Säule mit dem Puffer bis zur Grundlinie gewaschen worden war, wurde die Elution des Enzyms mit einem linearen Gradienten von Natriumchlorid bis zu 0,6M durchgeführt. Die Enzymaktivität wurde mit 0,25M Natriumchlorid eluiert. Die spezifische Aktivität in der Nähe des peaks wies ein fast beständiges Niveau auf. Die Reinigungsstufen der L-Sorboson-Dehydrogenase sind in der Tabelle 9 zusammengefasst.

## Tabelle 9

| Stufe | Total-protein (mg) | Total-aktivität (Einhei-ten) | spezifische Aktivität (m Einhei-ten/mg) | Ausbeute (%) |
|---|---|---|---|---|
| Cytosol | 6.600,0 | 324,3 | 49,1 | 100 |
| DEAE-Sepharose CL-6B | 338,3 | 239,7 | 708,5 | 73,9 |
| DEAE-Sephadex A-50 | 202,3 | 167,0 | 825,5 | 51,5 |
| Blue Sepharose CL-6B | 34,6 | 55,1 | 1592,5 | 17,0 |

(6) Reinheit des isolierten Enzyms

Zur Bestimmung der Reinheit des isolierten Enzyms wurde eine Elektrophorese mit Polyacrylamidgel (Trenngel; 7,5% Acrylamid; Elektrophoresebedingungen: 20 mA bei 4°C für 8 Stunden) durchgeführt. Das Enzym zeigte eine einzelne Bande, und es wurde durch 30-minütiges Anfärben mit 50 ml der Lösung von 0,05M Phosphatpuffer (pH-Wert 7,0) enthaltend 100 mg L-Sorboson, 5 mg Nitroblautetrazolium, 25 mg NAD und 5 mg Phenazinmethosulfat bestätigt, dass dieses Protein Enzymaktivität aufwies.

Die Elektrophorese mit Natriumdodecylsulfat (SDS) Polyacrylamidgel (Trenngel; 12,5% Acrylamid; 20 mA bei Raumtemperatur, 3 Stunden) diente zur Bestimmung des Reinheitsgrades und des Molekulargewichts des Enzyms. Das Enzym führte zu einer einzelnen Bande mit einem Molekulargewicht von 50.000 ± 5.000 (entsprechend vier Untereinheiten der oben unter (7) angegebenen Einheit; "Molekulargewicht"). Als Molekulargewichtstandards wurden Phosphorylase B (MW 92.500), Rinderserumalbumin (MW 66.200), Ovalbumin (MW 45.000), Carbonsäureanhydrase (MW 31.000), Trypsin-Inhibitor der Sojabohne (MW 21.500) und Lysozym (MW 14.400) verwendet.

(7) Identifikation des Reaktionsproduktes

Das Reaktionsgemisch enthaltend 0,2 ml des gereinigten Enzyms, 0,1 ml 0,5M Kaliumphosphatpuffer (pH-Wert 7,0), 0,1 ml 0,5M L-Sorboson, 0,4 ml 14mM NAD und Wasser in einem Endvolumen von 1 ml wurde 60 Minuten bei 30°C inkubiert. Das Reaktionsprodukt wurde durch Dünnschichtchromatographie und Hochdruckflüssigkeitschromatographie analysiert. Das Produkt wurde durch Vergleich mit einer authentischen Probe als 2-KGA identifiziert.

Beispiel 2

Nach der im Beispiel 1 beschriebenen Methode wurde L-Sorboson-Dehydrogenase aus den Stämmen Gluconobacter oxydans E-1 (FERM-P Nr. 8353), H-2 (FERM-P Nr. 8354) und L-8 (FERM-P Nr. 8355) isoliert und charakterisiert. Die Enzyme aus diesen Stämmen wiesen dieselben Eigenschaften wie diejenigen des Enzyms aus Gluconobacter oxydans UV-10 (FERM-P Nr. 8422) auf.

Beispiel 3

Das Reaktionsgemisch, enthaltend 100 ml zellfreien Extrakt von UV-10, FERM-P Nr. 8422 (Total-Enzymaktivität 115 Einheiten), hergestellt nach den oben beschriebenen Stufen (1) und (2) des Beispiels 1, 50 ml 0,5M Kaliumphosphatpuffer (pH-Wert 7,0), 50 ml 10% L-Sorboson-Lösung und 300 ml Wasser wurde bei 30°C unter leichtem Schütteln inkubiert. Es bildeten sich 700 mg/Stunde 2-Keto-L-gulonsäure.

**Patentansprüche**

1. Aus dem Mikroorganismsmus Gluconobacter oxydans oder Mutanten davon isolierbare L-Sorboson-dehydrogenase in reiner Form, die die Oxidation von L-Sorboson zu 2-Keto-L-gulonsäure in Gegenwart von Nicotinamidadenindinukleotid oder Nicotinamidadenindinukleotidphosphat als Coenzym katalysiert, gekennzeichnet durch die folgenden physiko-chemischen Eigenschaften:
   a) Optimaler pH-Wert: ca. 9
   b) Optimale Temperatur: ca. 60°C
   c) Molekulargewicht: 190.000 ± 20.000 (nämlich vier Untereinheiten mit je einem Molekulargewicht von 50.000 ± 5.000)
   d) Hemmung: durch $Cu^{2+}$ und N-Aethylmaleimid.

2. Verfahren zur Herstellung der L-Sorboson-Dehydrogenase gemäss Anspruch 1, dadurch gekennzeichnet, dass man einen zur Gattung Gluconobacter gehörenden Mikroorganismus oder eine Mutante davon, die zur Herstellung der neuen L-Sorboson-Dehydrogenase befähigt sind, kultiviert, das Zellmaterial zertrümmert, die L-Sorboson-Dehydrogenase aus zellfreiem Extrakt des zertrümmerten Zellmaterials, vorzugsweise aus der Cytosol-Fraktion des Zellmaterials, isoliert und die L-Sorboson-Dehydrogenase reinigt.

3. Verfahren zur Herstellung der neuen L-Sorboson-Dehydrogenase nach Anspruch 2, dadurch gekennzeichnet, dass der Mikroorganismus zur Gattung Gluconobacter oxydans gehört.

4. Verfahren zur Herstellung der neuen L-Sorboson-Dehydrogenase nach Anspruch 3, dadurch gekennzeichnet, dass der Mikroorganismus aus der Gruppe Gluconobacter oxydans UV-10 (FERM-P Nr. 8422), Gluconobacter oxydans E-1 (FERM-P Nr. 8353), Gluconobacter oxydans H-2 (FERM-P Nr. 8354) und Gluconobacter oxydans L-8 (FERM-P Nr. 8355) ausgewählt wird.

5. Verfahren zur Oxidation von L-Sorboson zu 2-KGA, dadurch gekennzeichnet, dass die Oxidation durch die in Anspruch 1 angegebene L-Sorboson-Dehydrogenase in Gegenwart von NAD oder NADP als Coenzym katalysiert wird.

**Claims**

1. L-sorbosone dehydrogenase in pure form, which is isolatable from the microorganism Gluconobacter oxydans or mutants thereof and which catalyzes the oxidation of L-sorbosone to 2-keto-L-gulonic acid in the presence of nicotinamide adenine dinucleotide or nicotinamide adenine dinucleotide phosphate as a coenzyme, characterized by the following physico-chemical properties:
   a) Optimum pH : about 9
   b) Optimun temperature : about 60°C
   c) Molecular weight : 190,000 ± 20,000 (namely four sub-units each having a molecular weight of 50,000 ± 5,000)
   d) Inhibition : by $Cu^{2+}$ and N-ethylmaleimide.

2. A process for the production of the L-sorbosone dehydrogenase in accordance with claim 1, characterized by cultivating a microorganism belonging to the genus Gluconobacter or a mutant thereof, which is capable of producing the novel L-sorbosone dehydrogenase, disrupting the cells, isolating the L-sorbosone dehydrogenase from cell-free extract of disrupted cells, preferably from the cytosol fraction of the cells, and purifying the L-sorbosone dehydrogenase.

3. A process for the production of the novel L-sorbosone dehydrogenase according to claim 2, characterized in that the microorganism belongs to the genus Gluconobacter oxydans.

4. A process for the production of the novel L-sorbosone dehydrogenase according to claim 3, characterized in that the microorganism is selected from the group Gluconobacter oxydans UV-10 (FERM-P No. 8422), Gluconobacter oxydans E-1 (FERM-P No. 8353), Gluconobacter oxydans H-2 (FERM-P No. 8354) and Gluconobacter oxydans L-8 (FERM-P No. 8355).

5. A process for the oxidation of L-sorbosone to 2-KGA, characterized in that the oxidation is catalyzed by the L-sorbosone dehydrogenase specified in claim 1 in the presence of NAD on NADP as a coenzyme.

**Revendications**

1. L-sorbosone-déshydrogénase sous forme pure, isolable à partir du microorganisme Gluconobacter oxydans ou de ses mutants, qui catalyse l'oxydation de la L-sorbosone en acide 2-céto-L-gulonique en présence de nicotinamide-adénine-dinucléotide ou de nicotinamide-adénine-dinucléotide-phosphate comme coenzyme, caractérisée par les propriétés physico-chimiques suivantes:
   a) pH optimal: environ 9
   b) température optimale: environ 60°C
   c) poids moléculaire: 190 000 ± 20 000 (à savoir quatre sous-unités ayant chacune un poids moléculaire de 50 000 ± 5000)
   d) inhibition: par $Cu^{2+}$ et le N-éthyl-maléimide.

2. Procédé de préparation de la L-sorbosone-déshydrogénase selon la revendication 1, caractérisé en ce qu'on cultive un microorganisme appartenant au genre Gluconobacter ou un de ses mutants qui sont apte à la préparation de la nouvelle L-sorbosone-déshydrogénase, en ce qu on brise la matière cellulaire, en ce qu'on isole la L-sorbosone-déshydrogénase à partir d'un extrait acellulaire de la matière cellulaire brisée, de préférence à partir de la fraction cytosol de la matière cellulaire, et en ce qu'on purifie la L-sorbosone-déshydrogénase.

3. Procédé de préparation de la nouvelle L-sorbosone-déshydrogénase selon la revendication 2, caractérisé en ce que le microorganisme appartient au genre Gluconobacter oxydans.

4. Procédé de préparation de la nouvelle L-sorbosone-déshydrogénase selon la revendication 3, caractérisé en ce que le microorganisme est choisi dans le groupe constitué par Gluconobacter oxydans UV-10 (FERM-P N° 8422), Gluconobacter oxydans E-1 (FEM-P N° 8353), Gluconobacter oxydans H-2 (FERM-P N° 8354) et Gluconobacter oxydans L-8 (FERM-P N° 8355).

5. Procédé d'oxydation de L-sorbosone en 2-KGA, caractérisé en ce que l'oxydation est catalysée par la L-sorbosone-déshydrogénase indiquée dans la revendication 1 en présence de NAD ou de NADP comme coenzyme.